Europäisches Patentamt

European Patent Office

Office européen des brevets

(11) Publication number : **0 585 058 A1**

(12) **EUROPEAN PATENT APPLICATION**

(21) Application number : 93306570.8

(22) Date of filing : 19.08.93

(51) Int. Cl.⁵ : **A61K 31/725**

(30) Priority : 25.08.92 GB 9218065
28.10.92 GB 9222655

(43) Date of publication of application :
02.03.94 Bulletin 94/09

(84) Designated Contracting States :
AT BE CH DE DK ES FR GB GR IE IT LI LU MC
NL PT SE

(71) Applicant : SCOTIA HOLDINGS PLC
Efamol House Woodbridge Meadows
Guildford Surrey GU1 1BA (GB)

(72) Inventor : Horrobin, David F. c/o Scotia
Pharmaceuticals Ltd
Efamol House, Woodbridge Meadows
Guildford, Surrey GU1 1BA (GB)
Inventor : Scott, Catherine A. c/o Scotia
Pharmaceuticals Ltd
Efamol House, Woodbridge Meadows
Guildford, Surrey GU1 1BA (GB)

(74) Representative : Sturt, Clifford Mark et al
J. Miller & Co. 34 Bedford Row Holborn
London WC1R 4JH (GB)

(54) **Pharmaceutical compositions containing fatty acids and heparin.**

(57)    Method of safe intravenous administration of fatty acids or salts or other derivatives of fatty acids or conjugated fatty acids, whereby heparin or related compound in a dose of 1,000 - 20,000 IU, preferably 3,000 - 10,000 IU or equivalent anticoagulant dose of heparin-like protein or peptide is administered subcutaneously or intravenously prior to infusion of the fatty acid, or where 1 - 100 IU/ml, preferably 10 to 20 IU/ml heparin or related compound, or equivalent anticoagulant dose of heparin-like protein or peptide is added to the infusion fluid carrying the fatty acid, or where both pretreatment with heparin-like protein or peptide and simultaneous administration are combined.
    This method is useful in the treatment of cancer, viral infections and other disorders, requiring maintenance of high plasma fatty acid levels.

EP 0 585 058 A1

The invention relates to a method for the safe intravenous administration of fatty acids, or salts or derivatives of fatty acids, or conjugated fatty acids.

Fatty acids have a number of therapeutic actions. This is particularly true of the n-6 and n-3 series essential fatty acids. Different fatty acids may have desirable effects in a wide range of disorders including disorders of inflammation, in cancer, in infections and particularly viral infections, in psychiatric disorders, in cardiovascular disorders, in diabetes, in immunological disorders, in renal disorders, in reproductive disorders, in osteoporosis and disorders of calcium metabolism, and in skin disorders. Possible uses for fatty acid therapy have been described in the literature (eg. in Horrobin DF, Reviews in Contemporary Pharmacotherapy, volume 1, number 1; in "Omega-6 Essential Fatty Acids", edited by DF Horrobin, Wiley-Liss, New York, 1990; and in many previous patent applications by the applicant).

Although many different fatty acids have been proposed in therapy of various types, particular interest applies to the n-6 and n-3 series fatty acids either when used as the free fatty acid or as various derivatives such as salts, esters, glycerides, amides and phospholipids. The lithium salts of the fatty acids are of particular interest because they have properties which make them of particular value in a variety of situations see for example USP 4 328 243, EP 0 068 854 (USP 4 386 072), EP 0 085 579, EP 0 234 733 (USP 4 753 964 and 4 810 497), EP 0 289 204, EP 0 305 097, and UK 2 222 080, which discuss the use of lithium with EFA's in different forms including salts of lithium itself.

The pathways of conversion of the main series of polyunsaturated fatty acids in the body are as in Table 1 below:

## TABLE 1

| n-6 | n-3 |
|---|---|
| 18:2 delta-9,12 (linoleic acid) | 18:3 delta-9,12,15 (alpha-linolenic acid) |

delta-6 desaturase

| 18:3 delta-6,9,12 (gamma-linolenic acid) | 18:4 delta-6,9,12,15 (stearidonic acid) |
|---|---|

elongation

| 20:3 delta-8,11,14 (dihomo-gamma-linolenic acid) | 20:4 delta-8,11,14,17 |
|---|---|

delta-5 desaturase

| 20:4 delta-5,8,11,14 (arachidonic acid) | 20:5 delta-5,8,11,14,17 ('eicosapentaenoic acid') |
|---|---|

elongation

| 22:4 delta-7,10,13,16 (adrenic acid) | 22:5 delta-7,10,13,16,19 |
|---|---|

delta-4 desaturase

| 22:5 delta-4,7,10,13,16 | 22:6 delta-4,7,10,13,16,19 ('docosahexaenoic acid') |
|---|---|

The above pathways are not normally reversible nor, in man, are n-3 and n-6 series acids interconvertible.

The acids, which in nature are of the all-cis configuration, are systematically named as derivatives of the corresponding octadecanoic, eicosanoic or docosanoic acids, e.g. delta-9,12-octadecadienoic acid or delta-4,7,10,13,16,19 docosahexaenoic acid, but numerical designations such as, correspondingly, 18:2 n-6 or 22:6 n-3 are convenient. Initials, for example, EPA for the 20:5 n-3 acid (eicosapentaenoic acid) or DHA for the 22:6 n-3 acid (docosahexaenoic acid), are also used but do not serve when n-3 and n-6 acids of the same chain length and degree of unsaturation exist as for example with the 22:5 acids. Trivial names in more or less common use in the n-6 series are as shown. Of the n-3 series only 18:3 n-3 has a commonly used trivial name, alpha-linolenic acid, though the name stearidonic acid is coming into use for the 18:4 n-3 acid and the names eicosapentaenoic acid and docosahexanenoic acid as such are also used. The alpha isomer of linolenic acid was characterised earlier than gamma-linolenic acid and reference in the literature simply to linolenic acid, especially in the earlier literature, is to the alpha-acid.

For some purposes it is desirable to get into the body the highest tolerable concentration of a fatty acid. This is particularly the case with cancer and with acute viral infections but can occur in many therapeutic situations. Certain fatty acids, in particular polyunsaturated fatty acids, and especially the n-6 and n-3 series fatty acids are able to kill cancer cells at concentrations which do not harm normal cells. Similar fatty acids are also able to kill viruses, in particular enveloped viruses, where they act in part by disrupting the lipid coat of the virus.

We and others have recently found that other fatty acids which have unsaturated double-bonds, notably conjugated fatty acids of chain length C-10 or greater, may also have selective actions in killing cancer cells at concentrations which do not harm normal cells (eg. Corneillius A.S., Yerram N.R., Cratz D.A., Spector A.A., Cancer Research 51: 6025-6030, 1991). Conjugation is defined as a series of alternating single or double bonds in the carbon chain (eg. Hopkins CY, Topics in Lipid Chemistry 3:37-87, 1972). The double bonds may be in either the cis or the trans configuration; this is in contrast to the situation with the essential fatty acids in which all the double bonds must be in the cis configuration. The conjugated fatty acids may have two or more doubly unsaturated bonds but it appears that those with three or four such bonds may be particularly effective. Many fatty acids with this conjugated characteristic are known (eg. Hopkins). Parinaric acid (18:4, 9 cis, 11 trans, 13 trans, 15 cis) and alpha-eleostearic acid (18:3, 9 cis, 11 trans, 13 trans) are good examples. As with essential fatty acids it seems probable that the efficacy of these fatty acids against cancer cells depends on their unusually easy oxidation.

In these and other serious disease situations it is desirable to achieve a high concentration of the fatty acid in the body fluids for as long a time as possible. This can best be achieved by intravenous administration. However, there is then a risk of thrombosis of peripheral veins when that route is used or, when a central venous catheter is used, of the subclavian or other central vein.

As a result of tests of various procedures, a regime has now been developed which allows fatty acids as such or as derivatives including lithium salts to be infused into peripheral or central veins without risk of thrombosis, making use of heparin, or related low molecular weight compounds based on heparin, or proteins or peptides with heparin-like properties.

Thus the invention provides a method of safely administering intravenously fatty acids or salts or other derivatives of fatty acids whereby heparin or related compound in a dose of 1,000 - 20,000 IU, preferably 3,000 - 10,000 IU, or equivalent anticoagulant dose of heparin-like protein or peptide is administered subcutaneously or intravenously prior to the fatty acid infusion or where 1 - 100 IU/ml, preferably 10 to 20 IU/ml heparin or related compound, or equivalent anticoagulant dose of heparin-like protein or peptide is added to the infusion fluid carrying the fatty acid, or where both pre-treatment of the patient with heparin or related compound or heparin-like protein or peptide and addition of heparin or related compound or heparin-like protein or peptide to the infusion fluid are combined.

The lithium salts are a particularly convenient way of administering the fatty acids since, by monitoring the plasma lithium level, the rate of infusion can be kept at a rate which prevents haemolysis caused by too high levels of the fatty acid. As described in copending European patent application (Agent's Ref. EP 3020701), haemolysis can usually be prevented if plasma lithium levels are kept below 0.4-0.5 millimolar in the first 48 hours and below 0.7 millimolar thereafter.

Also within the invention are methods of treatment of cancer and the other conditions referred to herein requiring maintenance of high plasma fatty acid levels wherein the above method is applied and, as such, fatty acid containing intravenous infusion media containing heparin or heparin-like protein or peptide as set out.

Further, according to the invention, heparin or related compound or heparin-like protein or peptide and fatty acids as specified above are used in the preparation of a two component medicament for use in safe intravenous administration of said acids, in particular for the treatment of cancer or any of the other conditions referred to herein requiring maintenance of high plasma fatty acid levels, wherein a first component comprises heparin or related compound in a dose of from 1,000 - 20,000 IU, preferably 3,000 - 10,000 IU, or equivalent anticoagulant dose of heparin-like protein or peptide, suitable for administration subcutaneously or intravenously prior to the administration of a second component which is an infusion fluid comprising the fatty acid, and optionally further comprises 1 - 100 IU/ml, preferably 10 to 20 IU/ml heparin or related compound, or equivalent anticoagulant dose of heparin-like protein or peptide.

The invention also relates to preparation of a medicament comprising acid and from 1-100 IU/ml preferably 10 to 20 IU/ml heparin or related compound or equivalent anticoagulant dose of heparin-like protein or peptide.

Normally, the acid and heparin containing components will be prepared and supplied together, but the present invention also embraces the addition of either the acid or heparin material to standard infusion media containing the other.

The following examples illustrate the application of the invention.

**EXAMPLES**

Example 1

Lithium gamma-linolenate, eicosapentaenoate, dihomo-gamma-linolenate, arachidonate, docosahexaenoate, docosapentaenoate (n-3 or n-6), adrenate, linoleate, stearidonate, alpha-linolenate, parinarate, alpha-eleostearate or other appropriate fatty acid lithium salt is made up at 5 - 500 mg/ml, preferably 50 - 200 mg/ml, in an appropriate solution such as 20% ethanol in water or 0.9% saline, in sterile ampoules. Such ampoules are then added to appropriate conventional intravenous fluids such as 0.9% saline, or other appropriate intravenous fluid to achieve a final concentration of 2 - 50 mg/ml, preferably 5 - 20 mg/ml in the fluid to be administered. Then there is added 1 - 100 international units (IU)/ml, preferably 10 - 20 IU/ml, of heparin or of a low molecular weight heparin or other peptide having an equivalent anticoagulant activity. This final intravenous fluid is then slowly administered intravenously to a patient requiring maintenance of high plasma fatty acid levels, in order to deliver 1-5000 mg/kg/day, preferably 10 - 1,000 mg/kg/day, more preferably 50 - 250 mg/kg/day of the lithium salt to the patient, previously given such heparin or peptide subcutaneously or intravenously at a dose of 1,000 - 20,000 IU preferably 3,000 - 10,000 IU, with little or no risk of thrombosis.

Example 2

The lithium salt in Example 1 is replaced by molar equivalent amount of free fatty acid or other forms as described herein, soluble in the intravenous fluid.

**Claims**

1. A method of safely administering intravenously fatty acids or salts or other derivatives of fatty acids or conjugated fatty acids whereby heparin or related compound in a dose of 1,000 - 20,000 IU, preferably 3,000 - 10,000 IU or equivalent anticoagulant dose of heparin-like protein or peptide is administered subcutaneously or intravenously prior to infusion of the fatty acid or where 1 - 100 IU/ml, preferably 10 to 20 IU/ml heparin or related compound, or equivalent anticoagulant dose of heparin-like protein or peptide is added to the infusion fluid carrying the fatty acid, or where both pre-treatment of the patient with heparin and addition of heparin to the infusion fluid are combined.

2. A method according to claim 1, wherein the fatty acid(s) are chosen from linoleic, gamma-linolenic, dihomo-gamma-linolenic, arachidonic, alpha-linolenic, stearidonic, eicosapentaenoic, docosapentaenoic, docosahexaenoic, parinaric or alpha-eleostearic acids.

3. A method of treatment of cancer or any of the other conditions referred to herein requiring maintenance of high plasma fatty acid levels, wherein the method of claim 1 or 2 is applied.

4. A fatty acid-containing intravenous infusion medium containing heparin or related compound or heparin-like protein or peptide as set out in claim 1 or 2.

5. Use of heparin or related compound or heparin-like protein or peptide and fatty acids or salts or other derivatives thereof or conjugated fatty acids in the preparation of a two component medicament for use in safe intravenous administation of said acids, in particular for the treatment of cancer or any of the other conditions referred to herein requiring maintenance of high plasma fatty acid levels, wherein a first component comprises heparin or related compound in a dose of from 1,000 - 20,000 IU, preferably 3,000 - 10,000 IU, or equivalent anticoagulant dose of heparin-like protein or peptide, suitable for administration subcutaneously or intravenously prior to the administration of a second component which is an infusion fluid comprising the fatty acid, and which optionally further comprises 1 - 100 IU/ml preferably 10 to 20 IU/ml heparin or related compound, or equivalent anticoagulant dose of heparin-like protein or peptide; or use of said acid and/or heparin or related compound, or heparin-like protein or peptide in, or for incorporation in, either such component separately, or for use with the other.

6. A use according to claim 5, wherein the fatty acid(s) are chosen from linoleic, gamma-linolenic, dihomo-gamma-linolenic, arachidonic, alpha-linolenic, stearidonic, eicosapentaenoic, docosapentaenoic, docosahexaenoic, parinaric or alpha-eleostearic acids.

7. Use of heparin or related compound or heparin-like protein or peptide and fatty acids or salts or other derivatives thereof or conjugated fatty acids in the preparation of a medicament for use in safe intravenous administration of said acids, in particular for the treatment of cancer or any of the other conditions referred to herein requiring maintenance of high plasma fatty acid levels, wherein the medicament comprises heparin or related compound in an amount of from 1-100 IU/ml, preferably 10 to 20 IU/ml, or equivalent anticoagulant dose of heparin-like protein or peptide.

8. A use according to claim 7, wherein the fatty acid(s) are chosen from linoleic, gamma-linolenic, dihomo-gamma-linolenic, arachidonic, alpha-linolenic, stearidonic, eicosapentaenoic, docosapentaenoic, docosahexaenoic, parinaric or alpha-eleostearic acids.

**European Patent Office**

# PARTIAL EUROPEAN SEARCH REPORT

which under Rule 45 of the European Patent Convention shall be considered, for the purposes of subsequent proceedings, as the European search report

Application Number

EP 93 30 6570

## DOCUMENTS CONSIDERED TO BE RELEVANT

| Category | Citation of document with indication, where appropriate, of relevant passages | Relevant to claim | CLASSIFICATION OF THE APPLICATION (Int.Cl.5) |
|---|---|---|---|
| X | DATABASE WPI Week 9218, Derwent Publications Ltd., London, GB; AN 92-150587 & WO-A-92 05797 (NAGAMITU, S.) 16 April 1992 * abstract * | 1,3-5,7 | A61K31/725 |
| X | DATABASE WPI Week 8624, Derwent Publications Ltd., London, GB; AN 86-154995 & SU-A-1 194 418 (KAZAN MED. INST.) 30 November 1985 * abstract * | 1,4,5,7 | |
| D,A | CANCER RESEARCH vol. 51, no. 22 , 15 November 1991 pages 6025 - 6030 CORNELLIUS, A.S. ET AL 'CYTOTOXIC EFFECT OF CIS-PARINARIC ACID IN CULTURED MALIGNANT CELLS' * the whole document * | 1-8 | |

TECHNICAL FIELDS SEARCHED (Int.Cl.5)

A61K

-/--

## INCOMPLETE SEARCH

The Search Division considers that the present European patent application does not comply with the provisions of the European Patent Convention to such an extent that it is not possible to carry out a meaningful search into the state of the art on the basis of some of the claims

Claims searched completely :

Claims searched incompletely :

Claims not searched :

Reason for the limitation of the search:

see sheet C

| Place of search | Date of completion of the search | Examiner |
|---|---|---|
| THE HAGUE | 11 November 1993 | MAIR, J |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

**European Patent Office**

**PARTIAL EUROPEAN SEARCH REPORT**

Application Number

EP 93 30 6570

## DOCUMENTS CONSIDERED TO BE RELEVANT

| Category | Citation of document with indication, where appropriate, of relevant passages | Relevant to claim |
|---|---|---|
| X | REVUE DE LIEGE<br>vol. 46, no. 3 , March 1991<br>pages 147 - 157<br>PHILIPPET, P. 'UTILISATION DES LIPIDES PAR VOIE INTRAVENEUSE EN PERIODE NEONATALE' | 1,2,4 |
| A | * the whole document *<br>* especially page 154, line 29-45 *<br>--- | 3,5-8 |
| X | JOURNAL OF PARENTERAL AND ENTERAL NUTRITION<br>vol. 4, no. 3 , 1980<br>pages 307 - 311<br>SOMANI, P. ET AL 'SAFFLOWER OIL EMULSION: SINGLE AND MULTIPLE INFUSIONS WITH OR WITHOUT ADDED HEPARIN IN NORMAL VOLUNTEERS' | 1,2,4 |
| A | * the whole document *<br>* especially page 311, line 10-31 *<br>----- | 3,5-8 |

**CLASSIFICATION OF THE APPLICATION (Int.Cl.5)**

**TECHNICAL FIELDS SEARCHED (Int.Cl.5)**

EPO FORM 1503 03.82 (P04C10)

EP 93 30 6570

-C_

Remark: Although claims 1-3
are formulated as a method of
treatment of the human/animal
body (Art. 52(4) EPC) the search
has been carried out and based on
the alleged effects of the
compound/composition